(19) Europäisches Patentamt
European Patent Office
Office européen des brevets

(11) **EP 3 888 622 A1**

(12) **EUROPEAN PATENT APPLICATION**

(43) Date of publication:
**06.10.2021 Bulletin 2021/40**

(51) Int Cl.:
*A61K 8/02* *(2006.01)*      *A61K 8/19* *(2006.01)*
*A61K 8/24* *(2006.01)*      *A61Q 11/00* *(2006.01)*

(21) Application number: **20167829.9**

(22) Date of filing: **02.04.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Unilever Global IP Ltd
Wirral, CH62 4ZD (GB)**

(72) Inventors:
• **ASHCROFT, Alexander Thomas
Cheshire, CH1 6LD (GB)**
• **LIMER, Adam John
Merseyside, CH6 3JW (GB)**

(74) Representative: **Tansley, Sally Elizabeth
Unilever PLC
Unilever Patent Group
Bronland 14
6708 WH Wageningen (NL)**

(54) **ORAL CARE SYSTEM**

(57)      A system for mitigating the sensitivity of teeth the system comprising a delivery device, the delivery device comprising:

i) a strip of an orally acceptable flexible material, having a strip surface capable of being applied to a tooth surface;
ii) a layer comprising a particulate calcium carbonate.

**Description**

**Field of the Invention**

The present invention relates to a system for treating tooth hypersensitivity.

**Background**

Tooth hypersensitivity is a common but painful condition affecting up to 20% of the adult population. Hypersensitive teeth may be sensitive to cold, heat, air or sugary foods.

The incidence of tooth hypersensitivity increases with age. Tooth hypersensitivity is believed to be related to the general increase in exposed root surfaces of teeth as a result of periodontal disease, toothbrush abrasion, or cyclic loading fatigue of the thin enamel near the dento-enamel junction. When root surfaces are exposed, dentinal tubules are also exposed. Dentinal tubules are naturally present in the dentinal layer of the tooth and they provide for an osmotic flow between the inner pulp region of the tooth and the outer root surfaces.

The currently accepted theory for tooth hypersensitivity is the hydrodynamic theory, based on the belief that open exposed dentinal tubules allow fluid flow through the tubules. This flow excites the nerve endings in the dental pulp. Clinical replicas of sensitive teeth viewed in a scanning electron microscope (SEM) reveal varying numbers of open or partially occluded dentinal tubules.

There are two categories of therapy for the treatment of tooth hypersensitivity based upon two modes of action. The first category, nerve-depolarising agents, are pharmaceutical agents such as potassium nitrate, which function by interfering with neural transduction of the pain stimulus.

The second category, known as occluding agents, function by physically blocking the exposed ends of the dentinal tubules, thereby reducing dentinal fluid movement and reducing the irritation associated with the shear stress described by the hydrodynamic theory.

An example of an occluding agent is found in US 5,270,031 which describes a tubule occluding desensitizer comprising a polyacrylic acid such as Carbopol® polymeric materials. Another tubule occluding composition is disclosed in US 5,374,417 which discloses a potassium salt of a synthetic anionic polymer, such as a polycarboxylate.

WO2014/023466, WO2014/023465 and WO2015/067422 disclose compositions for treating tooth hypersensitivity comprising calcium carbonate.

There is a continuing need for providing effective systems for delivering desensitizing agents to the teeth which can provide treatment and relief of hypersensitivity.

**Description of the Invention**

The present invention relates to a system for mitigating the sensitivity of teeth the system comprising a delivery device the delivery device comprising:

    i) a strip of an orally acceptable flexible material, having a strip surface capable of being applied to a tooth surface;
    ii) a layer comprising a particulate calcium carbonate.

**Detailed Description of the Invention**

The delivery device comprises calcium carbonate, preferably particulate calcium carbonate composed of primary particles.

By "primary particles" is meant individual particles, defined as the smallest discrete particles that can be seen by electron microscopy analysis (such as, for example, individual crystals).

The primary particles may associate under certain conditions to form larger secondary structures such as aggregates or agglomerates.

In one present invention, a suitable source of particulate calcium carbonate as defined above includes crystalline calcium carbonates in which the individual crystals have a prismatic morphology and an average size of 2 microns or less.

The term "crystalline" (in the context of particulate calcium carbonate) generally means a particulate calcium carbonate in which at least 50% by weight, preferably at least 75% by weight, more preferably at least 90% by weight, most preferably more than 95% by weight and ideally more than 99% by weight of the calcium carbonate particles are in the form of crystals.

The term "crystal" means an essentially fully dense solid composed of atoms arranged in an orderly repetitive array bounded by plane surfaces which are the external expression of internal structure.

Crystals may be identified and characterised by standard techniques known to those skilled in the art such as X-ray diffraction.

Crystalline forms of calcium carbonate are available naturally, or may be synthetically produced in three particular crystalline morphologies, calcite, aragonite, and less commonly found, vaterite. The vaterite form of calcium carbonate is metastable and irreversibly transforms into calcite and aragonite. There are many different polymorphs (crystal habits) for each of these crystalline forms. The calcite crystalline morphology is the most commonly used crystal form of calcium carbonate. Over 300 crystalline forms of calcite have been reported in the literature.

References to a "prismatic crystal morphology" (in the context of crystalline calcium carbonates) generally denote a crystalline calcium carbonate in which at least 50% by weight, preferably at least 75% by weight, more preferably at least 90% by weight, most preferably more than 95% by weight and ideally more than 99% by weight of the crystals are prismatic crystals. Prismatic crystals have a set of faces with parallel edges that are parallel to the vertical or "c"-axis direction. There can be three, four, six, eight or even twelve faces that can form a prism. For the purposes of the present invention, preferred prismatic crystals have a generally uniform, typically generally hexagonal cross-sectional shape.

References to a "scalenohedral crystal morphology" (in the context of crystalline calcium carbonates) generally denote a crystalline calcium carbonate in which at least 50% by weight, preferably at least 75% by weight, more preferably at least 90% by weight, most preferably more than 95% by weight and ideally more than 99% by weight of the crystals are scalenohedral crystals. Scalenohedral crystals have a set of faces that are inclined to the vertical or "c"-axis direction, each of which are scalene triangles (i.e. triangles whose three sides are unequal in length). For the purposes of the present invention, preferred scalenohedral crystals have a trigonal scalenohedral shape, with twelve scalene triangle faces.

Reference to acicular" in this context refers to the shape of the crystals. Usually, crystals grow in three directions, length, width and height. Some crystals however, have one or two preferred growth directions. Acicular crystals have a preferred crystal growth in one direction. Examples are crystals in the form of needles, rods, fibres, whiskers or columns and the like. For the purposes of the present invention, preferred acicular crystals are rod-like or needle-like with a generally uniform, typically generally circular, cross-sectional shape.

Crystal shape may be determined by standard techniques known to those skilled in the art such as scanning electron microscopy (SEM). SEM is an imaging and analysis technique based on the detection of electrons and X-rays that are emitted from a material when irradiated by a scanning electron beam. Imaging allows the user to distinguish between primary particles and aggregates or agglomerates.

Usually, crystals grow in three directions, length, width and height. Some crystals however, have one or two preferred growth directions. For the purposes of the present invention, preferred prismatic calcium carbonate crystals have a ratio between the length and the width of the crystal (the so-called aspect ratio) ranging from about 1:1 up to about 3:1 (length:width). The aspect ratio of a particle (e.g. a crystal) may typically be obtained from SEM photographs by averaging the ratio between the length and width of the particle, measured on at least 10 particles for 1 photograph.

Preferably the calcium carbonate for use in this invention has a prismatic, scalenohedral or acicular crystal morphology.

In one preferred aspect of the invention the calcium carbonate comprises primary particles which have a length of 2 microns or less.

For the purposes of the present invention, preferred prismatic calcium carbonate crystals have an average size which is less than 2 microns and generally ranges from about 0.1 to 2 microns, with a preferred size ranging from about 0.2 to about 1.5, more preferably from about 0.3 to about 1, most preferably from about 0.5 to 0.9 microns. Particle (e.g. crystal) size may be determined by standard techniques known to those skilled in the art such as sedimentation. Particle size values obtained from sedimentation techniques are normally expressed in terms of the equivalent spherical diameter (ESD), i.e. the diameter of a notional sphere having the same volume as the particle. The ESD value may be calculated based on the sedimentation rate of the particle in question as defined by Stokes' Law (Micromeritics SediGraph® 5100 Particle Size Analysis System Operator's Manual, V2.03, 1990). The sedimentation rate is measured using a finely collimated beam of low energy X-rays which pass through the sample cell to a detector. For a sample population of particles, the distribution of particle mass at various points in the cell affects the number of X-ray pulses reaching the detector. This X-ray pulse count is used to derive the particle size distribution expressed as the percent mass at given particle diameters. The median value of the ESD which can be derived from this distribution (i.e. the particular ESD value on the cumulative mass distribution curve at which 50 percent mass of the population has a higher ESD and 50 percent mass of the population has a lower ESD) is usually quoted as the average particle size of the sample population.

For the purposes of the present invention, preferred scalenohedral calcium carbonate crystals have an average size which is less than 2 microns and generally ranges from about 0.05 to about 1.5 microns, with a preferred size ranging from about 0.1 to about 1 micron, more preferably from about 0.15 to about 0.5 micron, most preferably from about 0.2 to 0.35 micron. A standard technique known to those skilled in the art for determining the average size of the individual crystals is air permeametry according to the Lea and Nurse method (standards NF X 11-601 and NF X 11602). This analytical technique determines the average particle size by measuring the pressure drop across a packed powder bed using a water manometer. The pressure drop is a function of the permeability of the packed bed. This is related to the surface area of the particles which is then transformed to an average size based upon the assumption that the particles are spherical. Average particle size (Dp) is obtained from the massic area (SM) derived from the Lea and Nurse method by making the assumptions that all the particles are spherical, non-porous and of equal diameter, and by neglecting contact surfaces between the particles. The relationship between Dp and SM is the following:

$$Dp = 6/(\rho SM)$$

where $\rho$ is the specific mass of the calcium carbonate. The average particle size (Dp) obtained in this way represents the average equivalent spherical diameter, or average ESD, where equivalent spherical diameter (ESD) is defined as the diameter of a notional sphere having the same volume as the particle.

For the purposes of the invention preferred acicular calcium carbonate is composed of a length of 2 microns or greater. Preferred acicular calcium carbonate crystals for use in the invention have a length ranging from 2 to 100 microns, more preferably from 10 to 30 microns and a width ranging from 0.1 to 4.0 microns, more preferably from 0.5 to 1.0 microns. A specific class of material suitable for use in the invention includes aragonite crystal form calcium carbonates such as those described for example in US 5,164,172.

The ratio between the length and the width of a crystal, the so-called aspect ratio, is higher than 1:1 for preferred acicular crystals. The higher the aspect ratio, the longer the crystal. For the purposes of the present invention the aspect ratio is preferably at least 2.5:1, more preferably at least 10:1. For example the aspect ratio may typically range from 2.5:1 to 200:1, and preferably ranges from 10:1 to 60:1, more preferably from 20:1 to 30:1.

Preferred calcium carbonate crystals, in particular scalenohedral prismatic calcium carbonate generally have a BET specific surface area higher than or equal to 0.1 m2/g, preferably higher than or equal to 1 m2/g, more preferably higher than or equal to 3 m2/g and most preferably higher than or equal to 4 m2/g. The calcium carbonate particles according to the invention generally have a BET specific surface area lower than or equal to 30 m2/g, preferably lower than or equal to 25 m2/g, more preferably lower than or equal to 20 m2/g, and most preferably lower than or equal to 15 m2/g. The BET specific surface area is usually measured according to the standard ISO 9277 which specifies the determination of the overall specific external and internal surface area of disperse or porous solids by measuring the amount of physically adsorbed gas according to the Brunauer, Emmett and Teller (BET) method.

Crystalline forms of calcium carbonate and suitable for use in the invention are available naturally, or may be produced by precipitation production technology. Typically, precipitated calcium carbonate is prepared by exposing calcium hydroxide slurry to a carbonation reaction. Control of the specific solution environment during the nucleation and growth of calcium carbonate governs the size and the shape of the crystals in the resulting precipitated calcium carbonate product.

A commercially available source of particulate prismatic calcium carbonate suitable for use in the invention is ViCALity® ALBAFIL® Precipitated Calcium Carbonate (PCC), ex Specialty Minerals Inc., Bethlehem, Pa. 18017.

A commercially available source of particulate scalenohedral calcium carbonate suitable for use in the invention is SOCAL® S2E Precipitated Calcium Carbonate (PCC), ex Solvay S.A.

Suitable acicular crystal form calcium carbonates for use in the invention are commercially available and include those marketed by Maruo Calcium Company Limited, Japan under the trade name WISCAL®.

Mixtures of any of the above described materials may also be used.

The device preferably comprises a phosphate source, more preferably the phosphate resides in a layer as part of the device. Preferably the same phosphate is used in the device and in the activator composition. Typically, the phosphate source makes up from 0.5 to 15%, and preferably, from 1 to 12%, and most preferably, from 2 to 9% by weight of the composition, based on total weight of the composition of the layer in which it is included and including all ranges subsumed therein.

The phosphate source that may be used in this invention is limited only to the extent that the same may be used in a composition suitable for use in an oral cavity. Illustrative examples of the types of phosphate source suitable for use in this invention include monosodium phosphate, sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate, mixtures thereof or the like. The phosphate source is preferably one which is water soluble.

In a preferred embodiment, the phosphate source used is one which results in an oral care composition having a pH from 5.5 to 8, preferably from 6 to 7.5, and most preferably, about neutral. In a most preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

Preferably the phosphate salt is in the same layer in the devices as the calcium carbonate.

The delivery device of the invention comprises a strip of an orally acceptable flexible material. The surface of the strip is capable of being applied to a tooth surface.

Preferably the device has an elongate shape of a length sufficient that when placed against the front surface of a user's teeth it extends across a plurality of teeth, and of sufficient width that it extends at least from the gumline of the teeth to the crowns of the teeth. The elongate shape is such that it minimises the need for subsequent applications and time to cover all the user's teeth.

In a further embodiment the strip is such that it can be sufficient that when placed against the front surface of a user's teeth it extends across a plurality of teeth, and of sufficient width that it extends at least from the front gumline of the teeth to the crowns of the teeth and to the gumline behind the user's teeth leading to total coverage of the teeth above the gumline.

Preferably the application device is rectangular in shape.

Preferably the strip/device has the enamel regeneration system deposited upon the strip surface thereof as a layer or more preferably multiple layers.

In a preferred embodiment the device is a strip comprising a plurality of layers.

Preferably the device comprises at least two layers; one layer comprises a non-dissolving backing film, a second layer comprises particulate calcium carbonate. More preferably the second layer further comprises a phosphate salt.

In one aspect of the invention the strip comprises third protective layer it is particularly preferred if the third protective layer comprises a water-soluble polymer.

It is preferable if the non-dissolving backing layer comprises materials such as polymers, natural and synthetic wovens, non-wovens, foil, paper, rubber, and combinations thereof. The strip of material may be a single layer of material or a laminate of more than one layer. Generally, the strip of material is substantially water impermeable. The material may be any type of polymer that is compatible with tooth whitening actives and is sufficiently flexible to be shaped and applied to the tooth surface. The material may comprise a single polymer or a mixtures of polymers. Suitable polymers include, but are not limited to, polyethylene, polypropylene, ethylvinylacetate, ethylvinyl alcohol, polyesters, polyamides, fluoroplastics and combinations thereof. Preferably, the material is polyethylene, in particular polyethylene terephthalate.

The strip of material is generally less than about 1 mm thick, preferably less than about 0.05 mm thick, and more preferably from about 0.001 to about 0.03 mm thick.

In one embodiment the device comprises a third layer. The third layer preferably comprises a water soluble polymer or polymers. Suitable water soluble polymers may be natural or synthetic. Suitable natural hydrocolloids and water soluble polymers include cellulosic material, a polysaccharide, a gum, a protein, a starch or a glucan. Examples include but are not limited to carboxymethyl cellulose, hydroxyl methyl cellulose, hydroxyethyl cellulose, hydroxypropyl cellulose, hydroxypropylmethyl cellulose, gum Arabic, xanthan gum, karaya gum, tragacanth, acacia, carrageenan, guar gum, locust bean gum, pectin, alginates, polydextrose, dextrin, dextran, amylose, chitin, chitosan, levan, elsinan, collagen, gelatin, zein, gluten, soy protein isolate, whey protein isolate, casein and pullan. Suitable water soluble synthetic polymers include but are not limited to poly(vinyl pyrrolidone), poly(vinyl alcohol), poly(acrylic acid), polyacrylates, Methylmethacrylate copolymer, carboxyvinyl polymer, polyethylene oxide and polyethylene glycol.

The advantage of having a water-soluble layer and a non-water soluble layer is that in the mouth the water-soluble layer dissolves in the saliva and the ingredients enclosed therein (the phosphate) can react with the ingredients in the second layer (silicate) to aid the in-situ regeneration of enamel on the teeth. Thus, the system provides an enamel regeneration system that can be used with the minimum of water, can be easily stored and is stable on storage.

Preferably the composition in one of the compositions within the layers of the invention comprises titanium dioxide. In particular, the layer comprising an adhesive such as polyethylene glycols or celluloses/hydroxyalkyl celluloses.

It is preferable if the thickness of the first layer is less than about 1 mm thick, preferably less than about 0.05 mm thick, and more preferably from about 0.001 to about 0.03 mm thick, second layer is less than about 1 mm thick, preferably less than about 0.5 mm thick, and more preferably from about 0.001 to about 0.3 mm thick and third layer (if present) is less than about 1 mm thick, preferably less than about 0.5 mm thick, and more preferably from about 0.001 to about 0.3 mm thick.

The activator composition preferably comprises an anhydrous base formulation in that the activator base comprises less less than 1wt% of the activator composition of water; preferably less than 0.5 wt%, more preferably less than 0.1 wt% of the total activator composition.

The activator composition preferably comprises a phosphate source. Preferably the phosphate system is as described above in the description for the device. More preferably the phosphate system used in the activator composition is identical in chemical consituents as the phosphate in the device.

Typically, the phosphate source makes up from 0.5 to 15%, and preferably, from 1 to 12%, and most preferably, from 2 to 9% by weight of the activator composition, based on total weight of the activator composition in which it is included and including all ranges subsumed therein.

It is preferable the activating system comprises a calcium source that is insoluble or slightly soluble in water.

Soluble means a source that dissolves in water to give a solution with a concentration of at least 0.1 moles per litre at room temperature. Insoluble means a source that dissolves in water to give a solution with a concentration of less than 0.001 moles per litre at room temperature. Slightly soluble, therefore, is defined to mean a source that dissolves in water to give a solution with a concentration of greater than 0.001 moles per litre at room temperature and less than 0.1 moles per litre at room temperature. Substantially free of, as used herein, means less than 1.5%, and preferably, less than 1.0%, and most preferably, from 0.0 to 0.75% by weight, based on total weight of the oral care composition, including all ranges subsumed therein. The calcium source suitable for use in this invention is limited only to the extent that the same may be used in an oral cavity. In a preferred embodiment, the calcium source employed is insoluble or slightly soluble in water, but most preferably, insoluble in water.

Illustrative examples of the types of calcium source that may be used in this invention include, for example, calcium phosphate (i.e., added), calcium gluconate, calcium oxide, calcium lactate, calcium carbonate, calcium hydroxide, calcium sulfate, calcium carboxymethyl cellulose, calcium alginate, calcium salts of citric acid, calcium silicate, mixtures thereof or the like. In a preferred embodiment the calcium source is calcium silicate. In a more preferred embodiment, the calcium silicate used is $(CaS_iO_3)$ whereby the same is made commercially available under the name Microcal ET by Ineos Silicas, Ltd.

In yet another preferred embodiment, the calcium source is insoluble calcium silicate, present as the composite material calcium oxide-silica $(CaO-SiO_2)$ as described in commonly-owned application Publication No. 2008/015117.

When a calcium silicate composite material is employed, the ratio of calcium to silicon (Ca:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise mono-calcium silicate, bi-calcium silicate, or tri-calcium silicate whereby ratios of calcium to silicon (Ca:Si) should be understood to be atom ratios.

The calcium source employed in this invention may be in a crystalline or amorphous state, and preferably, the same is in an amorphous state. In an often preferred embodiment, the calcium source is in a mesoporous state, i.e. the source is a material having pores with diameters from 1 nm to 50 microns. Mesoporous calcium silicate (MCS) is often preferred.

The MCS which may be used in this invention can be made by combining a calcium salt, a silica precursor like silicate and a structure-directing agent to yield a solid suitable for calcinating. A more detailed description of the process that may be conducted to make the MCS suitable for use in this invention is described in the aforementioned commonly-owned application, Publication No. WO 2008/015117.

The amount of calcium source in the composition is typically from 0.1 to 50%, and preferably, from 1 to 30%, and most preferably, from 5 to 20% by weight of the oral care composition based on total weight of the oral care composition and including all ranges subsumed therein.

A preferred form of calcium silicate is calcium silicate coated $TiO_2$. Examples of preferred forms of calcium silicate coated TO02 are disclosed in WO2012/031786 and WO2012/031785.

In one embodiment, the activator composition may comprise calcium carbonate as described above for the device.

The oral care activator composition described herein may comprise ingredients which are common in the art, such as:

- antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds such as 2,2' methylenebis-(4-chloro-6-bromophenol);
- anti-inflammatory agents such as ibuprofen, flurbiprofen, aspirin, indomethacin, etc.;
- anti-caries agents such as sodium trimetaphosphate and casein;
- plaque buffers such as urea, calcium lactate, calcium glycerophosphate and polyacrylates;
- vitamins such as Vitamins A, C and E;
- plant extracts;
- desensitizing agents, e.g. potassium citrate, potassium chloride, potassium tartrate, potassium bicarbonate, potassium oxalate, and potassium nitrate;
- anti-calculus agents, e.g. alkali-metal pyrophosphates, hypophosphite-containing polymers, organic phosphonates and phosphocitrates, etc;
- biomolecules, e.g. bacteriocins, antibodies, enzymes, etc ;
- flavors, e.g., peppermint and spearmint oils;
- proteinaceous materials such as collagen;
- preservatives;
- opacifying agents;
- coloring agents like FD&C blue, yellow and/or red dyes/colorants;

- pH-adjusting agents;
- sweetening agents;
- surfactants, such as anionic, cationic and zwitterionic or amphoteric surfactants (e.g., sodium lauryl sulfate, sodium dodecylbenzene sulfonate);
- particulate abrasive materials such as abrasive silicas, aluminas, calcium carbonates, zirconium silicate, polymethylmethacrylate, dicalciumphosphates, calcium pyrophosphates, hydroxyapatites, trimetaphosphates, insoluble hexametaphosphates as well as agglomerated particulate abrasive materials;
- fluoride sources like sodium fluoride, stannous fluoride, sodium monofluorophosphate, zinc ammonium fluoride, tin ammonium fluoride, calcium fluoride, cobalt ammonium fluoride or mixtures thereof;
- polymeric compounds which can enhance the delivery of active ingredients such as antimicrobial agents can also be included. Examples of such polymers are copolymers of polyvinylmethylether with maleic anhydride and other similar delivery enhancing polymers, e.g., those described in DE-A03,942,643; buffers and salts to buffer the pH and ionic strength of the oral care compositions; and
- other optional ingredients that may be included are, e.g., bleaching agents such as peroxy compound, e.g., potassium peroxydiphosphate, effervescing systems such as sodium bicarbonate/citric acid systems, color change systems, and the like.

Such ingredients common in the art typically and collectively make-up less than 20% by weight of the oral care composition, and preferably, from 0.0 to 15% by weight, and most preferably, from about 0.01 to about 12% by weight of the oral care composition, including all ranges subsumed therein.

Suitable carrier humectants are preferably used in the oral care system of the present invention, in particular the activation system and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. The carrier humectants should, in any case, be substantially free of water, and preferably, anhydrous. The same, for example, can be used in solid form, whereby glycerin is the preferred carrier humectant. Such carriers are particularly suitable in compositions used in the second layer.

The carrier humectant is used to take the balance of the compositions up to 100%, and the same may be present in the range of from 10 to 90% by weight of the oral care composition. Preferably, the carrier humectant makes up from 25 to 80%, and most preferably, from 45 to 70% by weight of the oral care composition, based on total weight of the oral care composition and including all ranges subsumed therein.

Mode of Use

The invention provides a method of de-sensitizing and remineralising teeth.

An activator composition can be present is applied to the strips of the invention. The activated strips as described above are applied to the surface of the teeth in sustained contact.

In the context of the present invention sustained contact means the product is left on the teeth for 1 to 60 minutes, preferably, about 5 to 45 minutes, more preferably 10 to 30 minutes before being removed.

Preferably the application of the oral care product of the invention is carried out once daily for a period of several consecutive days, in addition to a regular regime of tooth brushing (preferably at least twice daily).

Typically, use (for a period of about two weeks to one month) of the device with the oral care composition of the present invention will result in a new hydroxyapatite layer on teeth that is from 0.5 to 20 microns, and preferably, from 0.75 to 5 microns, including all ranges subsumed therein.

The invention will now be illustrated by the following non-limiting Examples:

**Examples**

Example 1

Formulation A:

| Ingredient | %w/w |
|---|---|
| Purified Water | To 100% |
| Titanium Dioxide | 0.500% |
| Glycerine | 2.000% |

(continued)

| Ingredient | %w/w |
|---|---|
| PEG-1500 NF (Polyethylene Glycol 1500) | 12.000% |
| White Beeswak, NF | 0.500% |
| PEG-40 Hydrogenated Castor Oil | 0.250% |
| Benzyl Alcohol EP/USP/NF | 0.001% |
| Phenoxyethanol | 0.001% |
| Hydroxyethyl Cellulose | 2.500% |
| Total | 100% |

Water was added to a mixing vessel followed by PEG-40 Hydrogenated Castor Oil, glycerine, PEG, beeswax, Benzyl Alcohol, Phenoxyethanol, titanium dioxide and mixed to disperse. Subsequently hydroxyethyl cellulose was slowly added to the vessel and mixed to disperse.

Formulation B:

| Ingredient | %w/w |
|---|---|
| Purified Water | To 100% |
| Glycerine | 16.000% |
| Hydroxyethyl Cellulose | 1.000% |
| Silica | 12.830% |
| Calcium Carbonate | 5% |
| Polyvinylpyrrolidone | 2.000% |
| Trisodium Phosphate | 1.750% |
| Monosodium Phosphate | 1.750% |
| Aroma | 0.250% |
| Benzyl Alcohol | 0.300% |
| Phenoxyethanol | 0.300% |
| Ethylhexylglycerin | 0.300% |
| Sodium Fluoride | 0.220% |
| PEG-40 Hydrogenated Castor Oil | 0.100% |
| Sodium Saccharin | 0.100% |
| Total | 100% |

Water was added to a mixing vessel followed by PEG-40 Hydrogenated Castor Oil, glycerine, ethylhexyl glycerine, phenoxyethanol, Benzyl Alcohol, flavour, monosodium phosphate, trisodium phosphate, sodium fluoride, sodium saccharine, silica and mixed to disperse. Subsequently hydroxyethyl cellulose and polyvinylpyrrolidone was slowly added to the vessel and mixed to disperse.

A film of formulation A was applied to a PET film and dried in an oven to a water content of 28-30wt%. On this film was placed a layer of non-woven Rayon fabric and a film of formulation B applied. Again, the sample was dried in an oven to a water content of 28-30wt%. The resulting structure has four layers, a PET film, a layer of formulation A, a layer or rayon and a layer of formulation B. The resulting structure can be pealed from the PET layer to give a three-layer structure.

The device is used together with the following activation solutions:
A pair of formulations were prepared by combining the following:

Table 3-Activating composition.

| Ingredient | Example 1C %W/W | Example 1D %W/W | Example 1E %W/W | Example 1F %W/W | Example 1G %W/W |
|---|---|---|---|---|---|
| Glycerol | To 100 | To 100 | To 100 | To 100 | To 100 |
| PEG 400 | 36.93 | 36.93 | 36.93 | 36.93 | 36.93 |
| Calcium Silicate | 15 | 15 | 15 | 15 | 15 |
| Omyacare S-100AV | 5 | 0 | 0 | 0 | 0 |
| OmyaCare HP900 | 0 | 5 | 0 | 0 | 0 |
| Socal S2E | 0 | 0 | 0 | 5 | 0 |
| Wiscal A | 0 | 0 | 0 | 0 | 5 |
| Sodium monofluorophosphate | 0.74 | 0.74 | 0.74 | 0.74 | 0.74 |
| Calcium Phosphate | 2 | 2 | 2 | 2 | 2 |
| Sodium Saccharine | 0.1 | 0.1 | 0.1 | 0.1 | 0.1 |
| Flavor | 0.3 | 0.3 | 0.3 | 0.3 | 0.3 |
| Anhydrous Mono Sodium Phosphate | 1 | 1 | 1 | 1 | 1 |
| Anhydrous Tri Sodium Phosphate | 2 | 2 | 2 | 2 | 2 |
| Total | 100 | 100 | 100 | 100 | 100 |

In use the activating solution was added to the device prior to application to the teeth.


**Claims**

1. A system for mitigating the sensitivity of teeth the system comprising a delivery device, the delivery device comprising:

   i) a strip of an orally acceptable flexible material, having a strip surface capable of being applied to a tooth surface;
   ii) a layer comprising a particulate calcium carbonate.

2. A system according to claim 1 in which the calcium carbonate comprises primary particles which are acicular and which have a length of 2 microns or greater.

3. A system according to claim 1 in which the calcium carbonate comprises primary particles which have a length of 2 microns or less.

4. A system according to claim 1 in which the calcium carbonate comprise of scalenohedral calcium carbonate crystals have an average size which is less than 2 microns.

5. A system according to claim 1 in which the calcium carbonate comprise of prismatic calcium carbonate crystals have an average size which is less than 2 microns.

6. A system according to any preceding claim in which the strip comprises a layer comprising a phosphate source.

7. A system according to any preceding claim in which the phosphate source comprises trisodium phosphate, sodium dihydrogen phosphate or mixtures thereof.

8. A system according to any preceding claim further comprising an activating composition.

9. A system according to any preceding claim in which the activating composition comprises a water insoluble and/or slightly soluble calcium source.

10. A system according to claim 9 in which the calcium source is calcium silicate.

11. A system according to any claim 9 or claim 10 in which the activating composition further comprises a phosphate source.

12. A system according to any one of claims 9 to 11 in which the phosphate source comprises trisodium phosphate, sodium dihydrogen phosphate or mixtures thereof.

13. A system according to any preceding claim in which the device comprises a plurality of layers.

14. A system according to claim 12, in which one layer of the device comprises a non-dissolving backing film.

15. A system according to claim 14 in which the non-dissolving backing film comprises polyethylene terephthalate.

16. A system according to any preceding claim in which the device has an elongate shape of a length sufficient that when placed against the front surface of a user's teeth it extends across a plurality of teeth, and of sufficient width that it extends at least from the gumline of the teeth to the crowns of the teeth.

17. A system according to any preceding claim in which the device is substantially rectangular.

Europäisches
Patentamt

European
Patent Office

Office européen
des brevets

## EUROPEAN SEARCH REPORT

Application Number

EP 20 16 7829

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/192924 A1 (UNILEVER PLC [GB]; UNILEVER NV [NL]; CONOPCO INC D/B/A UNILEVER [US]) 8 December 2016 (2016-12-08) * paragraphs [0001], [0009]; claims; examples * | 1-17 | INV. A61K8/02 A61K8/19 A61K8/24 A61Q11/00 |
| Y,D | WO 2014/023466 A1 (UNILEVER PLC [GB]; UNILEVER NV [NL]; CONOPCO INC DBA UNILEVER [US]) 13 February 2014 (2014-02-13) * claims; examples * | 1-17 | |
| Y,D | WO 2014/023465 A1 (UNILEVER PLC [GB]; UNILEVER NV [NL]; CONOPCO INC [US]) 13 February 2014 (2014-02-13) * examples * | 1-17 | |
| Y,D | WO 2012/031785 A2 (UNILEVER PLC [GB]; UNILEVER NV [NL] ET AL.) 15 March 2012 (2012-03-15) * claims; examples * | 1-17 | |

TECHNICAL FIELDS
SEARCHED      (IPC)

A61K
A61Q

The present search report has been drawn up for all claims

| Place of search | Date of completion of the search | Examiner |
|---|---|---|
| Munich | 17 September 2020 | Mitchell, Gemma |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                              

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 16 7829

This annex lists the patent family members relating to the patent documents cited in the above-mentioned European search report.
The members are as contained in the European Patent Office EDP file on
The European Patent Office is in no way liable for these particulars which are merely given for the purpose of information.

17-09-2020

| Patent document cited in search report | Publication date | Patent family member(s) | Publication date |
|---|---|---|---|
| WO 2016192924 A1 | 08-12-2016 | BR 112017023494 A2 | 24-07-2018 |
| | | CL 2017003086 A1 | 11-05-2018 |
| | | CN 107743386 A | 27-02-2018 |
| | | EA 201792165 A1 | 31-05-2018 |
| | | EP 3302349 A1 | 11-04-2018 |
| | | PH 12017502038 A1 | 11-04-2018 |
| | | US 2018125626 A1 | 10-05-2018 |
| | | WO 2016192924 A1 | 08-12-2016 |
| WO 2014023466 A1 | 13-02-2014 | BR 112015002531 A2 | 04-07-2017 |
| | | CL 2015000284 A1 | 28-08-2015 |
| | | CN 104684618 A | 03-06-2015 |
| | | EA 201590141 A1 | 30-07-2015 |
| | | EP 2882499 A1 | 17-06-2015 |
| | | PH 12015500239 A1 | 30-03-2015 |
| | | WO 2014023466 A1 | 13-02-2014 |
| WO 2014023465 A1 | 13-02-2014 | BR 112015002551 A2 | 04-07-2017 |
| | | CL 2015000283 A1 | 02-10-2015 |
| | | CN 104684619 A | 03-06-2015 |
| | | EA 201590140 A1 | 30-07-2015 |
| | | EP 2882500 A1 | 17-06-2015 |
| | | PH 12015500240 A1 | 30-03-2015 |
| | | WO 2014023465 A1 | 13-02-2014 |
| WO 2012031785 A2 | 15-03-2012 | BR 112013004354 A2 | 10-05-2016 |
| | | CN 103079645 A | 01-05-2013 |
| | | CN 107308010 A | 03-11-2017 |
| | | EP 2613850 A2 | 17-07-2013 |
| | | US 2013164359 A1 | 27-06-2013 |
| | | WO 2012031785 A2 | 15-03-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5270031 A
- US 5374417 A
- WO 2014023466 A
- WO 2014023465 A
- WO 2015067422 A

- US 5164172 A
- WO 2008015117 A
- WO 2012031786 A
- WO 2012031785 A
- DE 3942643 A0

**Non-patent literature cited in the description**

- **STOKES' LAW.** Micromeritics SediGraph® 5100 Particle Size Analysis System Operator's Manual. 1990, vol. 2.03